# EUROPEAN PATENT APPLICATION

(11) **EP 1 515 142 A1**
(43) Date of publication of application: **16.03.2005**
(21) Application number: 03020759.1
(22) Date of filing: 12.09.2003
(51) Int. Cl.: G01N 33/68, C07K 14/745

(54) **Mass spectrometry methods for direct phenotyping of factor XIIIA polymorphisms**

(71) Applicant: BioVisioN AG, 30625 Hannover (DE)
(72) Inventor: Tammen, Harald, Dr., 30449 Hannover (DE); Hess, Rüdiger, 30625 Hannover (DE); Lamping, Norbert, Dr., 30175 Hannover (DE); Möhring, Thomas, 22761 Hamburg (DE); Kellmann, Markus, 27243 Harpstedt (DE)
(74) Representative: Kröncke, Rolf, Dr.

(57) **Abstract**

The invention comprises novel factor XIIIA protein fragments and their use to determine the presence or absence of certain factor XIIIA polymorphisms. These polymorphisms are associated with diseases such as myocardial infarction, brain infarction, deep vein thrombosis, intracerebral hemorrhage and primary pulmonary embolism. The method of the invention measures the molecular mass of factor XIIIA proteins or protein fragments and uses these data to calculate differences of the molecular mass, indicating certain factor XIIIA polymorphism. Furthermore included in the invention are test kits, factor XIIIA protein fragments and antibodies binding to same.

## Description

### FIELD OF THE INVENTION

This invention relates to polymorphisms of the coagulation factor XIIIA protein of the clotting cascade. The invention relates to detection methods for this polymorphism as well as to protein fragments of factor XIIIA and antibodies binding to factor XIIIA protein or protein fragments.

### BACKGROUND OF THE INVENTION

In the final step of the clotting cascade coagulation factor XIIIA chain is activated by thrombin catalyzed removal of an activation peptide representing amino acids 1 to 37 of factor XIIIA. Active factor XIIIA generates intermolecular amide bonds between lysine and glutamine residues resulting in covalent cross linking of fibrin strands and conversion of soluble fibrin molecules into a stable insoluble clot. The involvement of factor XIIIA in cardio- and cerebrovascular diseases has been investigated recently and the presence of the Val34Leu mutation is known to correlate with a lower incidence of myocardial infarction and ischemic stroke but an increased risk for hemorrhagic stroke [1-5]. Other diseases whose occurrence is lowered if this polymorphism are deep vein thrombosis and pulmonary embolism. This mutation is localized in direct vicinity to the thrombin cleavage site and is thought to influence the activation process of this protein [1]. So far, the determination of the allelic genotype has been achieved by PCR-RFLP (Polymerase Chain Reaction-Restriction Fragment Length Polymorphism) [4] or allele specific PCR [6]. Although the reliability of this molecular biological techniques is proven, some restrictions remain due to the indirect analysis of a highly amplified descendant of the real target. Additionally, cautious handling of the sample material is essential to reduce the risk of contamination in diagnostic PCR.

### SUMMARY OF THE INVENTION

The invention provides a new method to detect factor XIIIA polymorphisms, especially the Val34Leu polymorphism, which is associated with decreased risks to certain diseases and increased risks to certain other diseases. For this reason a fast reliable detection method for factor XIIIA polymorphisms has a big medical impact, especially as factor XIIIA polymorphisms are quite common. The invention uses the determination of the masses of the whole factor XIIIA protein or factor XIIIA protein fragments to detect alterations in the amino acids sequence. In contrast to commonly used detection methods for factor XIIIA polymorphisms such as allele specific PCR or PCR-RFLP, which determine the genetic background of the individual tested, the invention claimed determines directly the phenotype, namely the presence or absence of certain factor XIIIA protein or protein fragments not just the DNA or RNA coding for factor XIIIA protein. Therefore the use of the invention gives more medical relevant information than standard genetic factor XIIIA polymorphism-testing and this information is much more closely related to the health status of the individual tested. The invention furthermore has the advantage, that no tedious, time and money consuming nucleotide-isolation procedures are necessary, as the invention directly uses biological samples such as blood or serum. These kind of samples are commonly obtained in routine medical diagnostic procedures as they are commonly used for many other medical tests such as cytokine, lipoprotein, hormone, etc. measurements. Furthermore factor XIIIA polymorphism tests known in the prior art such as PCR include an nucleic acid amplification step necessary to increase the amount of genetic material to test. This amplification step inherently bears the risk, that even the smallest amount of contamination of the test sample easily can result in false positive or negative results as the amplification step also amplifies contaminating nucleic acids. In contrast the invention claimed here directly measures the gene product, e.g. the factor XIIIA protein, without any amplification steps. Consequently small contaminations of the sample have no influence on the reliability of the test results. Further advantages of the invention over the prior art are the possibility to quantify the factor XIIIA protein present in the organism of the individual tested. Quantification of factor XIIIA RNA does not directly reflect the amount of factor XIIIA protein present and therefore is not as useful as the determination of the protein. It is also possible to determine if the individual is homo- or heterozygous for the polymorphism and the extend to which samples of heterozygous individuals contain factor XIIIA proteins of both types/alleles. It is not necessarily the case, that a heterozygous individual has 50 % of the normal factor XIIIA protein and 50% of the altered factor XIIIA protein. But for the medical impact of the factor XIIIA polymorphism the actual concentration of the different factor XIIIA proteins is important. Furthermore the invention claimed can be used to indirectly determine the factor XIIIA activation status of the individual tested. This is possible as one of the factor XIIIA peptides suitable for testing represents the activation peptide of factor XIIIA (Seq. ID 1 to 3), namely the peptide proteolytically released from factor XIIIA during the activation process of factor XIIIA, as described in "Background of the invention". This peptide is also known as coagulation factor activation peptide. Testing the activity of the clotting cascade increases the diagnostic uses of the invention even more, as many diseases such as inflammation, sepsis, cancer, thrombosis, clotting defects, autoimmune diseases, rheumatic diseases, etc. are associated with alterations in activity of the clotting cascade.

### DESCRIPTION OF THE PREFERRED EMBODIMENTS

A first aspect of the invention provides a method for the diagnosis of the presence or absence of a polymorphism of the factor XIIIA protein. Included are all sub forms of factor XIIIA such as factor XIIIA1, factor XIIIA2 and factor XIIIA3 and other sub forms known to date or discovered in the future. These polymorphisms include at least one amino acid altered (exchanged against a different one), deleted or inserted into the amino acid sequence of factor XIIIA. The amino acid sequence of factor XIIIA is noted under the accession no. NP_000120 and the nucleotide sequence of factor XIIIA is noted under the accession no. NM_000129. Both sequences are included in the sequence protocol under Seq. ID 16 (protein sequence) and Seq. ID 17 (nucleic acid sequence). Further sequences of factor XIIIA may be present in sequence data bases and are also included in the scope of the invention.

### Polymorphisms of factor XIIIA

Another embodiment of the invention is the determination of the molecular mass of proteins or protein fragments of certain know polymorphisms of factor XIIIA, especially of the Val35Leu polymorphism according to Seq.-ID. No. 16, which is commonly known as the Val34Leu polymorphism in the literature, in the following, the expression Val34Leu will be used for this polymorphism. Other polymorphisms are also suitable for use in the invention such as Pro565Leu, Leu589Gln, Thr551Ile, Va1651Ile, Arg682His and Gln652Glu according to Seq.ID No. 16. Other factor XIIIA polymorphisms known to date or in the future discovered can also be used within the invention. Such polymorphisms can be found in databases such as for example the Genecards™ database from the Weizmann Institute [7].

### Factor XIIIA fragments

Another embodiment of the invention is the detection of the mass of the complete factor XIIIA protein according to Seq. ID 16 or fragments thereof, preferably a fragment representing at least 8 consecutive amino acids of a factor XIIIA amino acid sequence.

### Modified factor XIIIA proteins and protein fragments

Postranslationally, chemically or enzymatically modified factor XIIIA proteins or protein fragments are also suitable for use in the inventions, especially N-terminal acetylated amino acid sequences. Seq. ID 1 and 2 were detected in the present study as N-terminal acetylated amino acid sequences. Other modified sequences may comprise modified/unusual amino acids, i.e. amino acids which do not belong to the 20 standard amino acids. Numerous examples of such amino acids are known in the art [8]. There are also numerous example of post-translational modifications known in the art such as phosphate- or sulphate-modifications, glycosylation, amidation, deamidation, pyroglutamate modifications, oxidized or amidated amino acid side chains etc.

### Mass spectrometry for mass determination

For detection of the molecular mass of factor XIIIA proteins or protein fragments all methods with an accuracy, which is good enough to distinguish single amino acid exchanges such as for example an exchange of valine against leucine, resulting in a mass difference of only 14 Dalton, are suitable. Preferably mass spectrometry is used for determination of small mass differences as this method is very sensitive and has a high resolution in a broad mass range. Even single amino acid exchanges within a complete protein sequence such as factor XIIIA, having a molecular weight of about 80,000 Dalton, can be detected by mass spectrometry, based on the mass differences resulting from the amino acid exchanges.

### Sample preparation prior to mass determination

The method of the invention optionally includes additional steps, which might improve or enable the determination of mass differences of factor XIIIA proteins or protein fragments. Such an additional optional step in the method is to perform a pre-fractionation of the sample by precipitation using preferably trichloroacetic acid or other precipitants such as ammonium sulfate, polyethylene glycol, acetone, ethanol etc. Other suitable precipitation methods are immune precipitations or precipitations initiated by alteration of physical factors such as temperature (heat precipitation). Fractions obtained using these methods can be further analyzed individually or in groups. Another sample preparation method is the use of extraction methods using liquid solvents. One suitable solvent is a mixture of an organic solvent such as polyethylene glycol and an aqueous salt solution. Other optional sample preparations methods are filtration or dialysis using membranes with distinct molecular weight cutoff values or membranes which are permeable preferably for certain substances based on their physicochemical properties such as hydrophobicity. Further suitable sample preparation methods are various kinds of chromatographic methods such as gel filtration, ion exchange, hydrophobicity or affinity chromatography, isoelectric focusing, capillary electrophoresis, gel electrophoresis, etc. The SELDI method also uses some kind of pre-fractionation by employing mass spectrometry targets, which are coated for example with an ion exchange matrix. These kind of method is also suitable for use in the invention presented here.

### Homozygous and heterozygous individuals

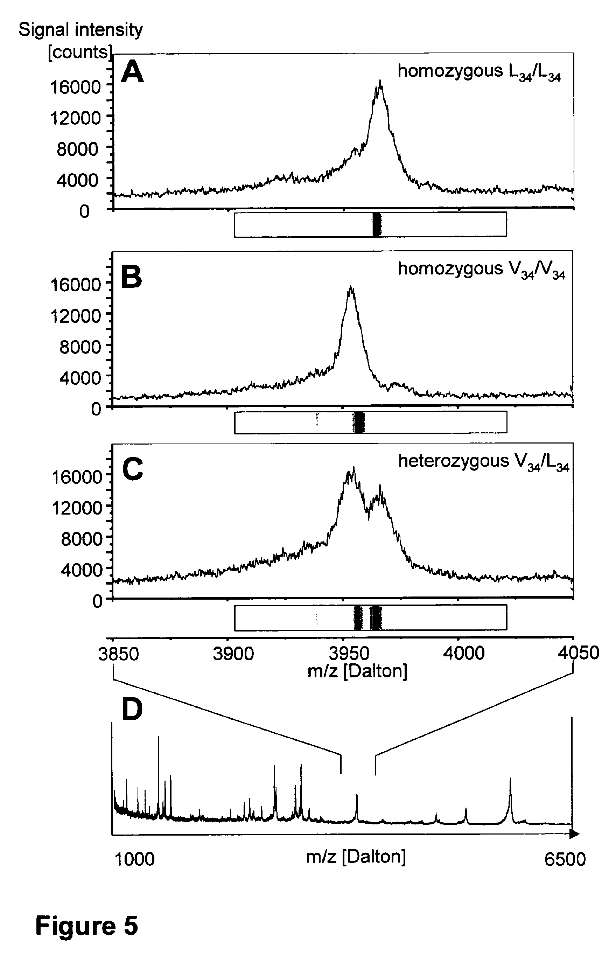

A preferred embodiment of the invention is the identification of homozygous and heterozygous individuals as shown in example 5 and in figure 5.

### Quantification of factor XIIIA

Mass spectrometry is suitable for quantification of distinct mass signals within complex samples and consequently another embodiment of the invention is the quantitative determination of factor XIIIA protein or protein fragments. This is of particular interest, as those results correspond to the concentrations of biological available factor XIIIA within the organism of the individual tested. Especially the concentration of the two different factor XIIIA variants present in heterozygous individuals can be determined individually for each variant even within the same measurement. Two separate measurements are also possible. In case these variants have different biological activity this information can be of particular medical interest.

### Indirect activity testing for factor XIIIA

Another embodiment of the invention is the use of the method of the invention to determine the amount of factor XIIIA activity present in the organism of the individual tested. This is possible as Seq. IDs 1 and 2 represent the so called 'activation peptide' of factor XIIIA, which is proteolytically released from factor XIIIA during factor XIIIA activation. Consequently the presence of the factor XIIIA protein fragment corresponding to Seq. IDs 1 and 2 and fragments of this factor XIIIA protein fragments such as Seq. IDs 4 to 15 indirectly reflect the activation of the clotting cascade within the organism of the individual tested.

### Diseases associated with factor XIIIA polymorphisms

A preferred embodiment of the invention is the prediction of a disease risk or the prognosis of an already existing disease, based on the presence or absence of homozygous or heterozygous polymorphisms within the amino acid sequence of factor XIIIA. Of particular interest is the factor XIIIA polymorphism at amino acid position 34 changing valine to leucine. This polymorphism results in an decreased risk for myocardial infarction, brain infarction and deep vein thrombosis [9-11]. Additionally this polymorphism increases the risk for intracerebral hemorrhage and primary pulmonary embolism [12, 13]. Other diseases are also connected to factor XIIIA polymorphisms, such as cancer or diabetes.

Another embodiment relates to the stratification of the therapy to be applied in individuals suffering from diseases related to factor XIIIA polymorphism as indicated herein.

### Factor XIIIA protein fragments and antibodies directed to these

A further embodiment of the invention are the newly identified factor XIIIA protein fragments of Seq. IDs 3 to 15 and the antibodies directed to these.

### Testkits

Further included in the invention are the use of factor XIIIA protein or protein fragments for use as standard in medical test kits, for example in mass spectrometric test kits to determine the presence of factor XIIIA polymorphisms or in test kits to determine the risk of suffering from certain factor XIIIA polymorphism-associated diseases such as myocardial infarction, brain infarction, deep vein thrombosis, intracerebral hemorrhage and primary pulmonary embolism. A preferred embodiment of the invention is the use of these factor XIIIA proteins or protein fragments in SELDI (surface enhanced Laser desorption ionisation) (Ciphergen Biosystems Inc., Fremont, CA, USA)test kits. Alternatively to mass spectrometric methods the newly identified factor XIIIA protein fragments containing a polymorphism can be used to produce polymorphism-specific antibodies. These antibodies or fragments of these antibodies and the corresponding factor XIIIA protein fragments bound by these antibodies can be used for the manufacture and for performing immunological test such as ELISA (enzyme linked immuno sorbent assay), RIAs (radio immuno assays), westernblots, SELDI-assays, plasmonresonance-assays, protein-chip assays, etc.

Thus, another embodiment of the invention is a method to detect the absence or presence of a factor XIIIA polymorphism by using polymorphism-specific antibodies.

### EXAMPLES

### Example 1: Sample preparation, deproteinizing of samples

The healthy adult men were enrolled into this study after they provided written informed consent and the local Ethics Committee at the Hanover Medical School approved the protocol. The blood samples were collected from the cubical vein into blood collection tubes (9 ml S- Monovette with clot activator, Sarstedt, Nürnberg, Germany) at one time point. The samples were made anonymous. After coagulation of the blood (approx. 1 h) the tubes were centrifuged for 10 min at 4°C at 2,000 x g. The serum was separated from the clot and stored at -80°C until further analysis. To reduce of protein load in the serum samples a trichloroacetic acid (TCA) precipitation was done. 0.5 ml serum were added to 1 ml ice cold distilled water. For protein precipitation 0.5 ml TCA (20 %) was added and the tubes were vigorously-mixed for 30 sec. After incubating for 30 min on ice, samples were centrifuged at 18,000 g for 30 min at 4°C. The supernatants were transferred into sample vials for use in factor XIIIA testing.

### Example 2. Chromatographic fractionation of serum samples for mass-spectrometric measurement of factor XIIIA protein and protein fragments

For the detection of factor XIIIA proteins and protein fragments from serum by use of mass spectrometry, it is necessary in this example to separate the peptide constituents. This sample pretreatment serves to concentrate the peptides of the invention and to remove components which may interfere with the measurement. The separation method carried out is a reverse phase chromatography. Various reverse phase chromatography resins and eluants are equally suitable for this. The separation of factor XIIIA-proteins and -protein fragments can be done using a C18 reverse phase chromatography column with the size of 4 mm x 250 mm supplied by Vydac. Mobile phases of the following composition were used: mobile phase A: 0.06% (v/v) trifluoroacetic acid, 4% (v/v) acetonitrile, mobile phase B: 0.05% (v/v) trifluoroacetic acid, 80% (v/v) acetonitrile. Chromatography took place at 33°C using an HP ChemStation 1100 supplied by Agilent Technologies with a micro flow cell supplied by Agilent Technologies. Human serum was used as sample. 330 µl of Serum were diluted with water to 1650 µl, the pH was adjusted to 2-3 using mobile phase A, the sample was centrifuged at 18,000x for 10 minutes and finally 1500 µl of the sample prepared in this way were loaded onto the chromatography column. The chromatography conditions were as follows: 5% mobile phase B at time 0 min, from time 1 to 45 min continuous increase in the mobile phase B concentration to 50%, from time 45 to 49 min continuous increase in the mobile phase B concentration to 100% and subsequently up to time 53 min constant 100% buffer B. Collection of 96 fractions each of 0.5 ml starts 10 minutes after the start of the chromatography.

### Example 3: Measurement of masses of peptides by means of MALDI mass spectrometry

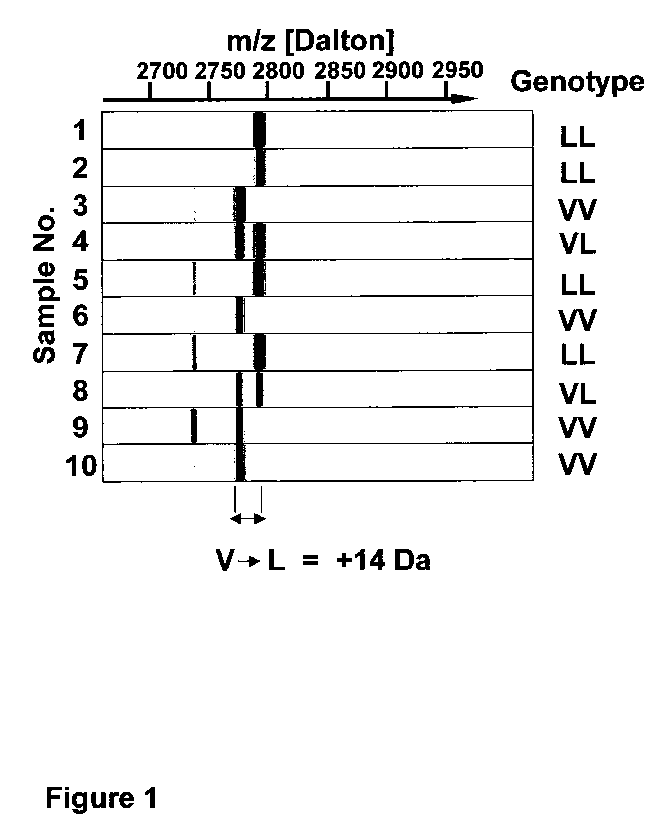

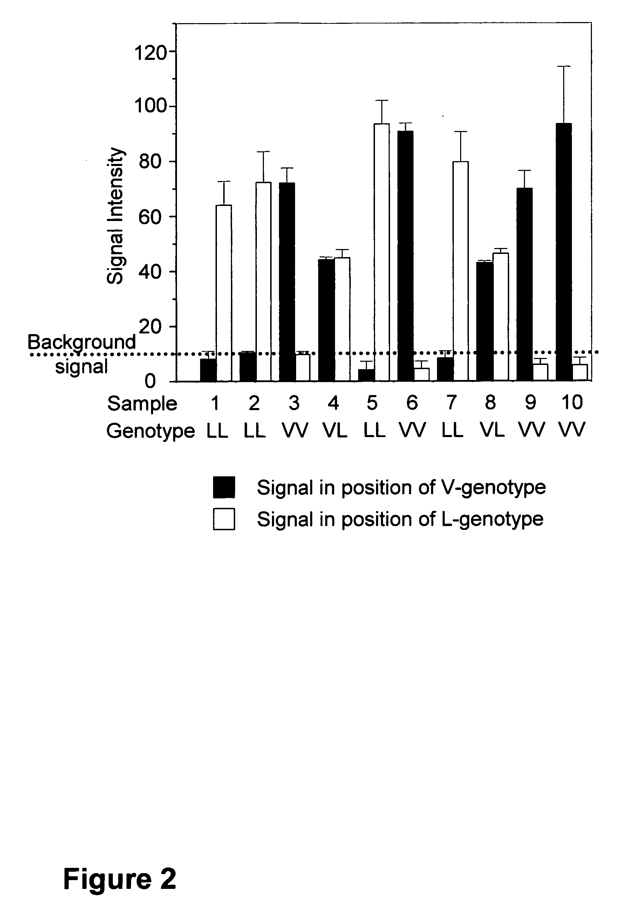

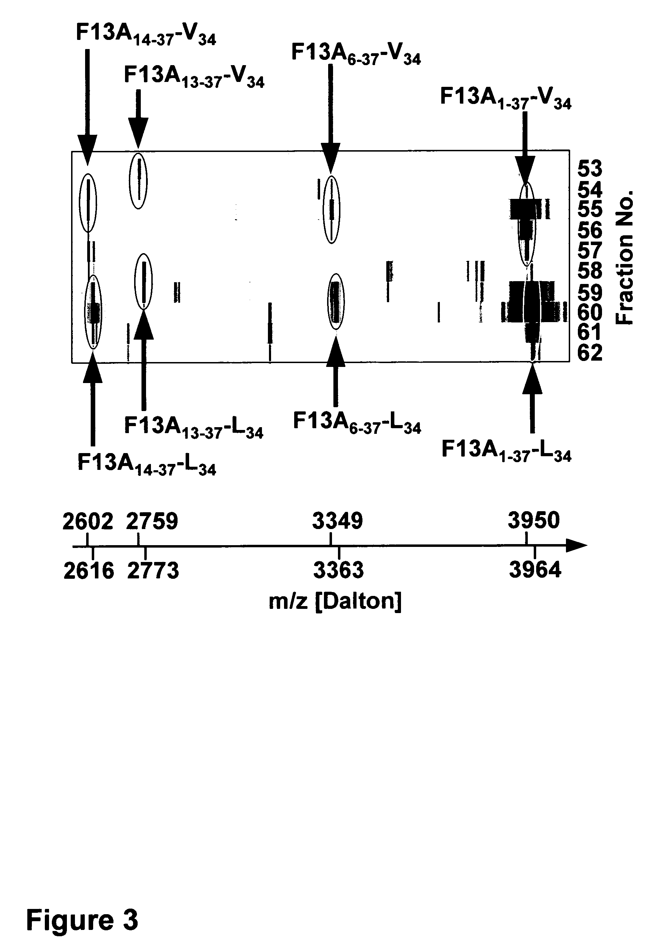

For mass analysis, typical positive ion spectra of peptides were produced in a MALDI-TOF (matrix-assisted laser desorption/ionization time-of-flight) mass spectrometer. Suitable MALDI-TOF mass spectrometers are manufactured by PerSeptive Biosystems Framingham (Voyager-DE, Voyager-DE PRO or Voyager-DE STR) or by Bruker Daltonik Bremen (BIFLEX). The samples are prepared by mixing them with a matrix substance which typically consists of an organic acid. Typical matrix substances suitable for peptides are 3,5-dimethoxy-4-hydroxycinnamic acid, α-cyano-4-hydroxycinnamic acid and 2,5-dihydroxybenzoic acid. A lyophilized equivalent obtained by reverse phase chromatography corresponding to 300 µl of human serum was used to measure the factor XIIIA protein fragments of the invention. The chromatographed lyophilized sample is dissolved in 15 µl of a matrix solution. This matrix solution contains, for example, 10 g/L α-cyano-4-hydroxycinnamic acid and 10 g/L L(-)fucose dissolved in a solvent mixture consisting of acetonitrile, water, trifluoroacetic acid and acetone in the ratio 49:49:1:1 by volume. 0.3 µl of this solution is transferred to a MALDI carrier plate, and the dried sample is analyzed in a Voyager-DE STR MALDI mass spectrometer from PerSeptive Biosystems. The measurement takes place in linear mode with delayed extraction™. Figure 1 shows the mass spectra of all 10 individuals tested in this study in the mass rage from 2700 to 2950 Dalton. For each sample there is shown the mass spectrum in gel-style view of the factor XIIIA protein fragment representing amino acids 6-37. The signal intensity is depicted as color intensity and the mass of the signal as the position of the band on the x-axis. The average signal intensity of the mass signal of a 5 Dalton mass window around the mass of the particular factor XIIIA protein fragment according to the sequence of amino acids 6 to 37 of the factor XIIIA protein from all 10 individuals tested are shown in figure 2. The signal intensity for both variants of the factor XIIIA protein fragment are present in heterozygote individuals and both signals are lower than the corresponding signals in homozygote individuals. There were additional factor XIIIA protein fragments present in serum, which could be determined by their masses using mass spectrometry. The corresponding mass signals, shown in gel-view style, are shown in figure 3. The x-axis at the bottom of figure 3 indicates the molecular masses determined experimentally for these factor XIIIA fragments. The following table gives an overview of factor XIIIA protein fragments of this invention:

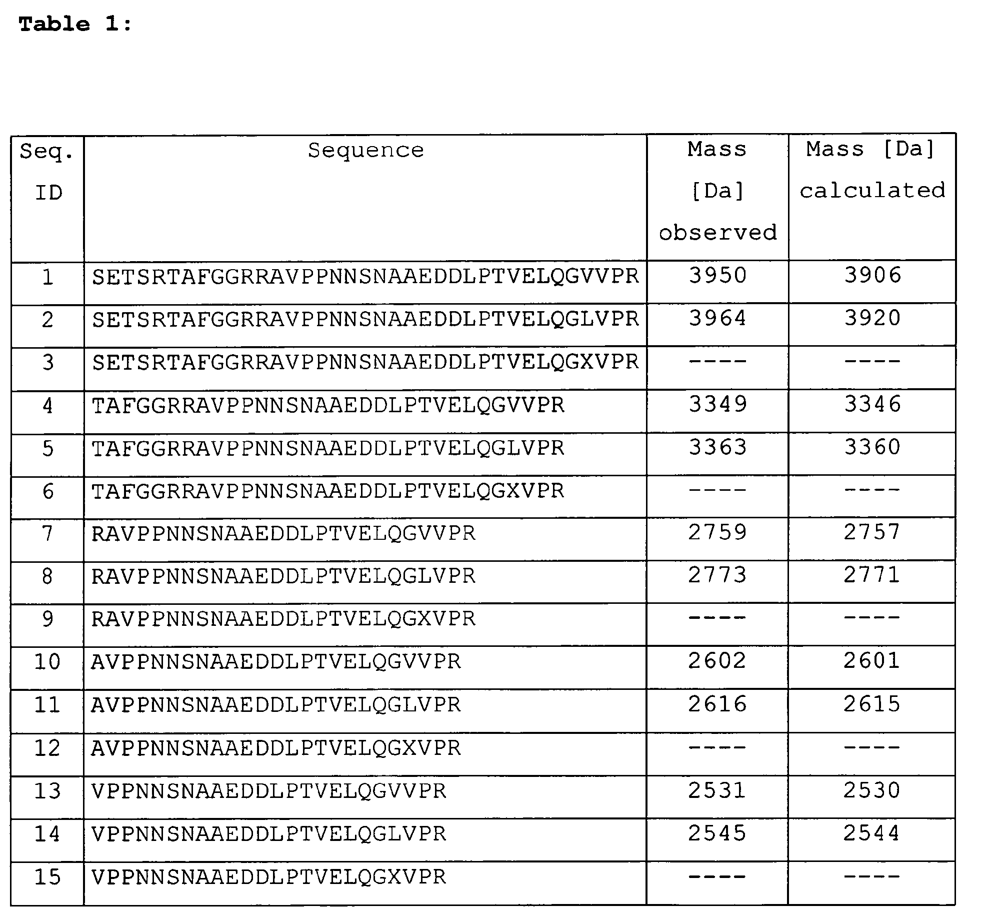

Sequence IDs 3, 6, 9, 12 and 15 contain the amino acid redidue "X" on position 34 of the corresponding factor XIIIA sequence. X represents any amino acid except valine or leucine. The experimentally observed mass values slightly differ from the calculated mass as a result of experimental error. The calculated mass values were calculated using the software GPMAV, version 4.02 and were rounded to full Dalton values. Seq. IDs 13 and 14 were not confirmed by sequencing but were calculated on basis of the mass differences observed, as compared to Seq. ID 10 and 11 which was exactly the mass of an alanine amino acid residue.

### Example 4: Identification of factor XIIIA protein fragments by mass spectrometry

For quantification of the factor XIIIA protein fragments of the invention it is necessary to ensure that the mass signals to be analyzed in fact relate to the factor XIIIA protein fragments of the invention.

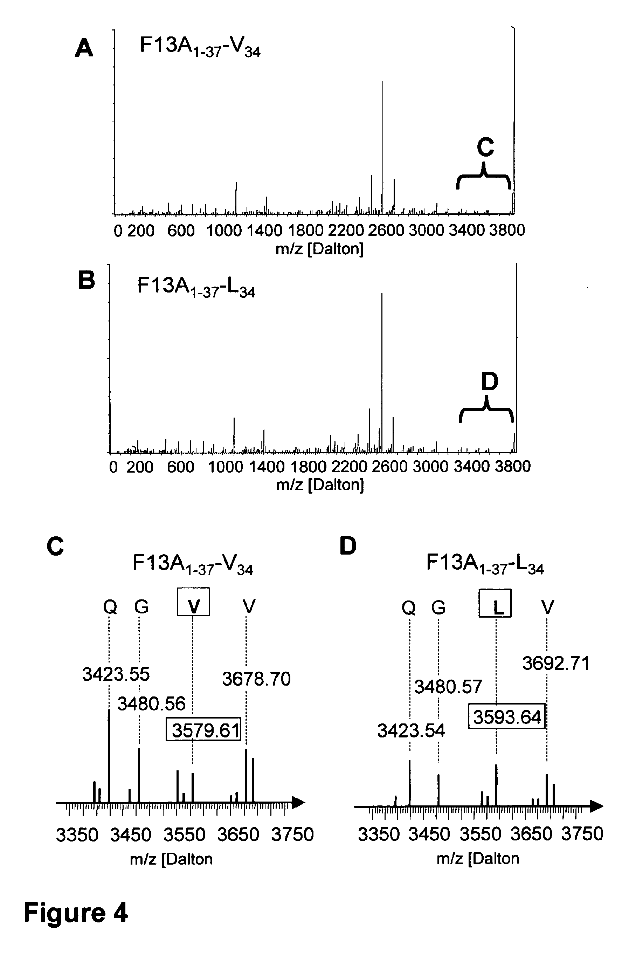

The peptides of the invention are sequenced for example using nanoSpray-MS/MS [14]. This entails a factor XIIIA protein fragment ion being selected in the mass spectrometer on the basis of its specific m/z (mass/charge) value in a manner known to the skilled worker. This selected ion is then fragmented by supplying collision energy with an collision gas, e.g. helium or nitrogen, and the resulting fragments of the factor XIIIA protein fragment are detected in the mass spectrometer in an integrated analysis unit, and corresponding m/z values are determined (principle of tandem mass spectrometry) [15]. The fragmentation behavior of peptides makes unambiguous identification of the factor XIIIA protein fragments of the invention possible. If the accuracy of mass is, for example, 50 ppm it is possible to use computer-assisted search methods [16] for search sequences of factor XIIIA protein fragments in sequence databases into which the sequence of a factor XIIIA-protein has been entered. In this specific case, the mass-spectrometric analysis took place with a Quadrupol-TOF Instrument, QStar-Pulsar model from Applied Biosystems-Sciex, USA. Examples of MS/MS fragment spectra are shown in Figure 4.

### Example 5: Masspectrometric measurement of serum samples without prior chromatographic fractionation of the sample

For sample preparation prior to MALDI-TOF-MS ZipTip devices containing C18 reverse phase matrix (Millipore) were rinsed with acetonitrile and equilibrated three times with 5 µL 0.1 % trifluoroacetic acid (TFA), 5 % acetonitrile, 94.9 % water. Subsequently 5 µL of deproteinized (as described in example 1) serum, equivalent to 1.25 µL serum, were aspirated and the device was washed three times with 5 µL of 0.1 % TFA, 5 % acetonitrile, 94.9 % water. Peptides were eluted directly onto a MALDI-Target using 1 µL of a mixture of alpha-cyano-4-hydroxycinnamic acid (matrix) and 6-desoxy-1-galactose (co-matrix) dissolved in a mixture of water, acetonitrile, acetone and TFA (41.45 %, 58 %, 0.5 %, 0.05 %). MALDI analysis was performed using a Voyager STR mass spectrometer (Applied Biosystems). Examples of measurements of factor XIIIA-protein fragments of the invention are shown in Figure 5. Figure 5 D shows an overview of a mass spectrum in the mass range from 1000 to 6500 Dalton, whereas figure 5A to 5C show details of such mass spectra. Figure 5A shows the mass peak of the peak representing the fragment of factor XIIIA from amino acid 1 to 37, which is identical to Seq. ID 1 (homozygous individual with valine in position 34), figure 5B shows the mass peak of the peak representing the fragment of factor XIIIA from amino acid 1 to 37, which is identical to Seq. ID 2 (homozygous individual with leucine in position 34) and figure 5C shows the two different mass peaks representing two identical fragments of factor XIIIA from amino acid 1 to 37, which differ only in amino acid position 34 (valine or leucine), corresponding to Seq. IDs 1 and 2 from a sample of a heterozygous individual with leucine or valine in position 34. The mass difference between valine and leucine (= 14 Dalton) results in the slightly different molecular masses of the two different peaks shown in figure 5A and 5B or shown in combination (heterozygous individual) in figure 5C. Alternatively mass spectra can also be shown in gel-view style. In gel-view style the signal intensity is depicted as line/band of distinct color intensity, whereas the molecular mass is depicted as the position of the line/band resulting in pictures which look very similar to pictures of stained SDS polyacrylamide gels (lower panel of figure 5A, 5B and 5C). Homozygous individuals result in one factor XIIIA protein fragment band (figure 5A and 5B lower panels) whereas heterozygous individuals result in two factor XIIIA protein fragment bands (figure 5C lower panel).

### DESCRIPTION OF THE FIGURES

### Figure 1

Figure 1 shows the mass spectra of all 10 individuals tested in this study in the mass rage from 2700 to 2950 Dalton. For each sample there is shown the mass spectrum in gel-style view of the factor XIIIA protein fragment representing amino acids 6-37. The signal intensity is depicted as color intensity and the mass of the signal as the position of the band on the x-axis. The sample no. is shown on the left hand side and the genotype of the individual tested (as determined by independent standard genetic methods) is shown on the right hand side with "LL" indicating that both alleles of factor XIIIA have a leucine in position 34, "VV" indicating, that both alleles of factor XIIIA have a valine in position 34 and "VL" indicating that the individual is heterozygous with one allele having a leucine and the second allele having a valine on position 34 of factor XIIIA. The mass shift form the "LL" phenotype to the "VV" phenotype is 14 Dalton (indicated at the bottom of figure 1) representing the mass difference between valine (= 117 Dalton) and leucine (= 131 Dalton).

### Figure 2

Figure 2 shows the signal intensity of a factor XIIIA protein fragment representing amino acids 6-37 of Factor XIIIA at the mass position of the "LL" genotype and the "VV" genotype. The signal intensities shown are the average signal intensities in a mass range of 5 Dalton around the genotype specific mass. The dotted line indicates the background signal, e.g. the "VV"-signal in an individual with "LL" genotype or the "LL" signal measured in a sample of an individual with "VV" genotype. The "LL"- and the "VV"-signals of heterozygous individuals (sample 4 and 8) show reduced signal intensities.

### Figure 3

Figure 3 shows mass spectra in gel-style view, obtained using a sample of a heterozygous individual. Depicted is a mass range from about 2600 to 4000 Dalton of the fractions 53 to 62. The identity of all indicated bands was confirmed by sequencing as described in example 4. The following factor XIIIA protein fragments were identified: amino acid 1 to 37, amino acid 6 to 37, amino acid 13 to 37 and amino acid 14 to 37. All of these sequences were found with valine and with leucine at position 34. The corresponding masses determined experimentally are shown on the x-axis at the bottom of figure 3.

### Figure 4

Figure 4 depicts the mass spectrometric sequencing of the factor XIIIA protein fragment representing amino acids 1 to 37. Panel A and B show the mass fragment spectra of the "VV"-genotype (panel A) and the "LL" genotype (panel B). Panel D and C show a detail of the mass fragment spectra of panel A and B, showing the mass range from 3350 to 3750 Dalton. Certain peaks of panel C and D are labeled with their respective molecular mass in Dalton. In addition these fragment masses are labeled with the last amino acid of the corresponding fragment of the factor XIIIA protein fragment in the one letter amino acid code. The boxed amino acid residue in panel C and D represents the amino acid in position 34 of factor XIIIA, which allows to distinguish between the "VV"-and the "LL"-genotype. In panel C the mass of the fragment with valine on position 34 has a molecular mass of 3579.61 Dalton, whereas the mass of the fragment with leucine in position 34 in panel D has a molecular mass of 3593.64. The difference between these two masses is 14 Dalton which is the mass difference between valine and leucine. The next bigger fragments in panel C and D have the molecular masses of 3678.70 and 3692.71. As these fragments also differ in position 34 from each other (valine exchanged against leucine) their masses also differ in 14 Dalton.

### Figure 5

Figure 5 shows the results of three measurements using serum which was not fractionated into 96 fractions prior to mass spectrometry as described in example 5. Mass spectra of the factor XIIIA protein fragment representing amino acid 1 to 37 from a sample of an individual of the "LL" genotype (panel A), from an individual of the "VV"-genotype (panel B) and from a heterozygous individual with the "VL"-genotype (panel C) are shown. Panel D shows a mass spectrum of a broader mass range from 1000 to 6500 Dalton as an overview, whereas panel A to C show only a very small mass range from 3850 to 4050 Dalton. Figure 5 shows that the "VV"-, the "LL" and the "VL"-genotype easily can be distinguished, even if the sample measured is not fractionated into 96 fractions.

The headings in this document are intended merely to provide structure to the text. They are not intended to limit or restrict the matters described. All the examples are intended to characterize the concept of the invention in more detail but are not intended to restrict the equivalence range of the invention.

In case this patent contains a term or phrase which is not clear to the skilled worker of that particular field or which is not clear in the context of the description provided by this patent the following references shall provide the definition for that term or phrase. In case there are different definitions for one term found in different references, the definition found in the reference citated first in the following list should be taken. The following references are cited for this purpose:
- The Merck Manual of Diagnosis and Therapy [17]
- Molecular Cloning - A Laboratory Manual [18]
- Current Protocols in Immunology [19]
- Current Protocols in Protein Science [20]
- Current Protocols in Pharmacology [21]
- Current Protocols in Cell Biology [22]

### CITATIONS

1.Undas, A., W.J. Sydor, B. K., J. Musial, K.G. Mann, and A. Szczeklik. 2003. Aspirin alters the cardioprotective effects of the factor XIII Val34Leu polymorphism. *Circulation.* 107:17-20.
2.Incorvaia, C., C. Costagliola, F. Parmeggian, D. Gemmati, G.L. Scapoli, and A. Sebastiani. 2002. Recurrent episodes of spontaneous subconjunctival hemorrhage in patients with factor XIII Val34Leu mutation. *Am J Ophthalmol*. 134:927-929.
3.Kohler, H.P., and V. Schroder. 2002. Role of coagulation factor XIII in cardio- and cerebrovasular diseases. *Hamostaseologie.* 22:53-58.
4.Balogh, I., G. Szoke, L. Karpati, U. Wartiovaara, E. Katona, I. Komaromi, G. Haramura, G. Pfliegler, H. Mikkola, and L. Muszbek. 2000. Val34Leu polymorphism of plasma factor XIII: biochemistry and epidemiology in familial thrombophilia. *Blood.* 96:2479-2486.
5.Aleksic, N., C. Ahn, Y.W. Wang, H. Juneja, A.R. Folsom, E. Boerwinkle, and K. Wu. 2002. Factor XIIIA Val34Leu polymorphism does not predict risk of coronary heart disease: The Atherosclerosis Risk in Communities (ARIC) Study. *Arterioscler Thromb Vasc Biol*. 22:348-52.
6.Henry, M., P.E. Morange, I. Canavy, M.C. Alessi, and I. Juhan-Vague. 1999. Rapid detection of factor XIII Val34Leu by allele specific PCR. *Thromb Haemost.* 81:463.
7.Rebhan, M., V. Chalifa-Caspi, J. Prilusky, and D. Lancet. 1997. GeneCards: encyclopedia for genes, proteins and diseases. *Weizmann Institute of Science, Bioinformatics Unit and Genome Center (Rehovot, Israel), URL: http:*//*bioinformatics.weizmann.ac.il*/*cards.*
8.Garavelli, J.S., Z. Hou, N. Pattabiraman, and R.M. Stephens. 2001. The RESID Database of protein structure modifications and the NRL-3D Sequence-Structure Database. *Nucleic Acids Res.* 29:199-201.
9.Kohler, H.P., M.H. Stickland, N. Ossei-Gerning, A. Carter, H. Mikkola, and G. P.J. 1998. Association of a common polymorphism in the factor XIII gene with myocardial infarction. *Thromb Haemost.* 79:8-13.
10.Elbaz, A., O. Poirier, S. Canaple, F. Chedru, F. Cambien, and P. Amarenco. 2000. The association between the Val34Leu polymorphism in the factor XIII gene and brain infarction. Blood. 95:586-591.
11.Catto, A.J., H.P. Kohler, J. Coore, M.W. Mansfield, M.H. Stickland, and P.J. Grant. 1999. Association of a common polymorphism in the factor XIII gene with venous thrombosis. Blood. 93:906-908.
12.Catto, A.J., r.H.P. Kohle, S. Bannan, M. Stickland, A. Carter, and G. P.J. 1998. Factor XIII Val 34 Leu: a novel association with primary intracerebral hemorrhage. Stroke. 29:813-816.
13.Ehrenforth, S., M. Krause, F.H. Herrmann, K. Wulff, B. Zwinge, and I. Scharrer. 2000. Pathogenisis of primary pulmonary embolism: impat of the factor XIII: Val34Leu polymorphism. *Haemostasis.* 30:30.
14.Wilm, M., and M. Mann. 1996. Analytical properties of the nanoelectrospray ion source. *Anal Chem.* 68:1-8.
15.Papayannopoulos, I.A. 1995. The interpretation of collision-induced dissociation tandem mass spectra of peptides. *Mass Spectrom Rev*:49-73.
16.Perkins, D.N., D.J. Pappin, D.M. Creasy, and J.S. Cottrell. 1999. Probability-based protein identification by searching sequence databases using mass spectrometry data. *Electrophoresis.* 20:3551-67.
17.Beers, M.H., and R. Berkow. 1999. The Merck Manual of Diagnosis and Therapy. *Merck Research Laboratories, Whitehous Station, NJ, USA.* 17th Edition.
18.Sambrook, J., and D.W. Russell. 2001. Molecular Cloning - A Laboratory Manual. *Cold Spring Harbor Laboratory Press, Cold Spring Harbor, NY, USA*. 3rd Edition.
19.Coligan, J.E., A.M. Kruisbeek, D.H. Margulies, and E.M. Shevach. 2002. Current Protocols in Immunology. *John Wiley & Son, Inc. , Hoboken, NJ, USA.*
20.Coligan, J.E., B.M. Dunn, H.L. Ploegh, D.W. Speicher, and P.T. Wingfield. 2002. Current Protocols in Protein Science. *John Wiley & Son, Inc., Hoboken, NJ, USA.*
21.Enna, S.J., M. Williams, J.W. Ferkany, T. Kenakin, R.D. Porsolt, and J.P. Sullivan. 2002. Current Protocols in Pharmacology. *John Wiley & Son, Inc., Hoboken, NJ, USA.*
22.Bonifacino, J.S., M. Dasso, J. Lippincott-Schwartz, J.B. Harford, and K.M. Yamada. 2002. Current Protocols in Cell Biology. *John Wiley & Son, Inc., Hoboken, NJ, USA.*

## Claims

1. A method to detect the absence or presence of a polymorphism of factor XIIIA by detecting the presence or absence of at least one amino acid substitution, deletion or insertion present in factor XIIIA or a fragment thereof as compared to Seq ID No. 16 or a corresponding fragment thereof by determining the mass of proteins or protein fragments of factor XIIIA.

2. A method to detect the absence or presence of a polymorphism of factor XIIIA by detecting the presence or absence of at least one amino acid substitution, deletion or insertion present in factor XIIIA or a fragment thereof by
a) measuring the mass of the protein or a protein fragment of factor XIIIA
b) calculating the mass of the corresponding protein or protein fragment of factor XIIIA using Seq. ID 16
c) determine if the masses determined in a) and calculated in b) differ from each other
d) regarding a mass difference between the masses of a) and b) as indicating a factor XIIIA polymorphism

3. A method according to claim 1 or 2 for the diagnosis of a polymorphism of factor XIIIA, wherein an amino acid substitution at amino acid position 34 of human factor XIIIA according to Seq ID No 16, preferably a valine/leucine substitution, is detected.

4. The method of any one of claims 1 to 3, wherein the factor XIIIA protein or protein fragment detected is
a) the complete factor XIIIA protein according to Seq. ID No 16 or a fragment thereof,
b) at least one sequence chosen from Seq. 1 to 15, or
c) a postranslationally modified sequence according to a) to b)

5. The method of any one of the preceding claims, **characterized in** using mass spectrometry for determination of the mass of the factor XIIIA protein or protein fragments thereof.

6. The method of any one of the preceding claims using a sample which is diluted, concentrated or fractionated prior to analysis, preferably using precipitation, filtration, chromatography or affinity-isolation methods.

7. The use of the method any one of claims 1 to 6 to distinguish homozygote and heterozygote individuals.

8. The use of the method of any one of claims 1 to 6 to quantify factor XIIIA protein present in homozygote or heterozygote individuals.

9. The use of the method of any one of claims 1 to 6 to determine the extend of factor XIIIA activation in the sample.

10. The use of the method of any one of claims 1 to 6 to determine a predisposition of an individual to at least one disease chosen from myocardial infarction, brain infarction, deep vein thrombosis, intracerebral hemorrhage, primary pulmonary embolism, cancer and diabetes.

11. Factor XIIIA fragments according to Seq. ID 3 to 15.

12. Antibodies or antibody-fragments or antibody-fusion proteins binding to factor XIIIA fragments according to Seq. ID 3 to 15.

13. A test kit for a method as claimed in any one of claims 1 to 6, preferably a method using mass spectrometry.

14. Use of a factor XIIIA protein or protein fragment as a standard in a test kit as claimed in claim 13.

15. Use of a substance as claimed in claim 11 or an antibody as claimed in claim 12 for the manufacture of a test kit preferably an ELISA, a RIA, a Westernblot, a SELDI-assay, a plasmonresonance-assay or a protein-chip assay.


SEQUENCE LISTING
<110>  BioVisioN AG
<120>  Use of mass determination of factor XIIIA protein or factor XIIIA
peptides for direct phenotyping of factor XIIIA polymorphisms
<130>  FXIIIA
<160>  17
<170>  PatentIn version 3.1
<210>  1
<211>  37
<212>  PRT
<213>  Homo sapiens

<400>  1

Ser Glu Thr Ser Arg Thr Ala Phe Gly Gly Arg Arg Ala Val Pro Pro
1               5                   10                  15

Asn Asn Ser Asn Ala Ala Glu Asp Asp Leu Pro Thr Val Glu Leu Gln
            20                  25                  30

Gly Val Val Pro Arg
        35

<210>  2
<211>  37
<212>  PRT
<213>  Homo sapiens

<400>  2

Ser Glu Thr Ser Arg Thr Ala Phe Gly Gly Arg Arg Ala Val Pro Pro
1               5                   10                  15

Asn Asn Ser Asn Ala Ala Glu Asp Asp Leu Pro Thr Val Glu Leu Gln
            20                  25                  30

Gly Leu Val Pro Arg
        35

<210>  3
<211>  37
<212>  PRT
<213>  Homo sapiens<

<220>
<221>  MISC_FEATURE
<222>  (34)..(34)
<223>  x can represent any amino acid except Val or Leu

<400>  3

Ser Glu Thr Ser Arg Thr Ala Phe Gly Gly Arg Arg Ala Val Pro Pro
1               5                   10                  15

Asn Asn Ser Asn Ala Ala Glu Asp Asp Leu Pro Thr Val Glu Leu Gln
            20                  25                  30

Gly Xaa Val Pro Arg
        35

<210>  4
<211>  32
<212>  PRT
<213>  Homo sapiens

<400>  4

Thr Ala Phe Gly Gly Arg Arg Ala Val Pro Pro Asn Asn Ser Asn Ala
1               5                   10                  15

Ala Glu Asp Asp Leu Pro Thr Val Glu Leu Gln Gly Val Val Pro Arg
            20                  25                  30

<210>  5
<211>  32
<212>  PRT
<213>  Homo sapiens

<400>  5

Thr Ala Phe Gly Gly Arg Arg Ala Val Pro Pro Asn Asn Ser Asn Ala
1               5                   10                  15

Ala Glu Asp Asp Leu Pro Thr Val Glu Leu Gln Gly Leu Val Pro Arg
            20                  25                  30

<210>  6
<211>  32
<212>  PRT
<213>  Homo sapiens

<220>
<221>  MISC_FEATURE
<222>  (29)..(29)
<223>  x can represent any amino acid except Val or Leu

<400>  6

Thr Ala Phe Gly Gly Arg Arg Ala Val Pro Pro Asn Asn Ser Asn Ala
1               5                   10                  15

Ala Glu Asp Asp Leu Pro Thr Val Glu Leu Gln Gly Xaa Val Pro Arg
            20                  25                  30

<210>  7
<211>  26
<212>  PRT
<213>  Homo sapiens

<400>  7

Arg Ala Val Pro Pro Asn Asn Ser Asn Ala Ala Glu Asp Asp Leu Pro
1               5                   10                  15

Thr Val Glu Leu Gln Gly Val Val Pro Arg
            20                  25

<210>  8

<211>  26
<212>  PRT
<213>  Homo sapiens

<400>  8

Arg Ala Val Pro Pro Asn Asn Ser Asn Ala Ala Glu Asp Asp Leu Pro
1               5                   10                  15

Thr Val Glu Leu Gln Gly Leu Val Pro Arg
            20                  25

<210>  9
<211>  26
<212>  PRT
<213>  Homo sapiens

<220>
<221>  MISC_FEATURE
<222>  (23)..(23)
<223>  x can represent any amino acid except Val or Leu

<400>  9

Arg Ala Val Pro Pro Asn Asn Ser Asn Ala Ala Glu Asp Asp Leu Pro
1               5                   10                  15

Thr Val Glu Leu Gln Gly Xaa Val Pro Arg
            20                  25

<210>  10
<211>  25
<212>  PRT
<213>  Homo sapiens

<400>  10

Ala Val Pro Pro Asn Asn Ser Asn Ala Ala Glu Asp Asp Leu Pro Thr
1               5                   10                  15

Val Glu Leu Gln Gly Val Val Pro Arg
            20                  25

<210>  11
<211>  25
<212>  PRT
<213>  Homo sapiens

<400>  11

Ala Val Pro Pro Asn Asn Ser Asn Ala Ala Glu Asp Asp Leu Pro Thr
1               5                   10                  15

Val Glu Leu Gln Gly Leu Val Pro Arg
            20                  25

<210>  12
<211>  25
<212>  PRT
<213>  Homo sapiens

<220>
<221> MISC_FEATURE
<222> (22)..(22)
<223> x can represent any amino acid except Val or Leu

<400> 12

Ala Val Pro Pro Asn Asn Ser Asn Ala Ala Glu Asp Asp Leu Pro Thr
1               5                   10                  15

Val Glu Leu Gln Gly Xaa Val Pro Arg
            20                  25

<210> 13
<211> 24
<212> PRT
<213> Homo sapiens

<400> 13

Val Pro Pro Asn Asn Ser Asn Ala Ala Glu Asp Asp Leu Pro Thr Val
1               5                   10                  15

Glu Leu Gln Gly Val Val Pro Arg
            20

<210> 14
<211> 24
<212> PRT
<213> Homo sapiens

<400> 14

Val Pro Pro Asn Asn Ser Asn Ala Ala Glu Asp Asp Leu Pro Thr Val
1               5                   10                  15

Glu Leu Gln Gly Leu Val Pro Arg
            20

<210> 15
<211> 24
<212> PRT
<213> Homo sapiens

<220>
<221> MISC_FEATURE
<222> (21)..(21)
<223> x can represent any amino acid except Val or Leu

<400> 15

Val Pro Pro Asn Asn Ser Asn Ala Ala Glu Asp Asp Leu Pro Thr Val
1               5                   10                  15

Glu Leu Gln Gly Xaa Val Pro Arg
            20

<210> 16
<211> 732

<212>  PRT
<213>  Homo sapiens

<400>  16

Met Ser Glu Thr Ser Arg Thr Ala Phe Gly Gly Arg Arg Ala Val Pro
1               5                   10                  15

Pro Asn Asn Ser Asn Ala Ala Glu Asp Asp Leu Pro Thr Val Glu Leu
            20                  25                  30

Gln Gly Val Val Pro Arg Gly Val Asn Leu Gln Glu Phe Leu Asn Val
        35                  40                  45

Thr Ser Val His Leu Phe Lys Glu Arg Trp Asp Thr Asn Lys Val Asp
    50                  55                  60

His His Thr Asp Lys Tyr Glu Asn Asn Lys Leu Ile Val Arg Arg Gly
65                  70                  75                  80

Gln Ser Phe Tyr Val Gln Ile Asp Leu Ser Arg Pro Tyr Asp Pro Arg
                85                  90                  95

Arg Asp Leu Phe Arg Val Glu Tyr Val Ile Gly Arg Tyr Pro Gln Glu
            100                 105                 110

Asn Lys Gly Thr Tyr Ile Pro Val Pro Ile Val Ser Glu Leu Gln Ser
        115                 120                 125

Gly Lys Trp Gly Ala Lys Ile Val Met Arg Glu Asp Arg Ser Val Arg
    130                 135                 140

Leu Ser Ile Gln Ser Ser Pro Lys Cys Ile Val Gly Lys Phe Arg Met
145                 150                 155                 160

Tyr Val Ala Val Trp Thr Pro Tyr Gly Val Leu Arg Thr Ser Arg Asn
                165                 170                 175

Pro Glu Thr Asp Thr Tyr Ile Leu Phe Asn Pro Trp Cys Glu Asp Asp
            180                 185                 190

Ala Val Tyr Leu Asp Asn Glu Lys Glu Arg Glu Glu Tyr Val Leu Asn
        195                 200                 205

Asp Ile Gly Val Ile Phe Tyr Gly Glu Val Asn Asp Ile Lys Thr Arg
    210                 215                 220

Ser Trp Ser Tyr Gly Gln Phe Glu Asp Gly Ile Leu Asp Thr Cys Leu
225                 230                 235                 240

Tyr Val Met Asp Arg Ala Gln Met Asp Leu Ser Gly Arg Gly Asn Pro
                245                 250                 255

Ile Lys Val Ser Arg Val Gly Ser Ala Met Val Asn Ala Lys Asp Asp
            260                 265                 270

Glu Gly Val Leu Val Gly Ser Trp Asp Asn Ile Tyr Ala Tyr Gly Val
        275                 280                 285

Pro Pro Ser Ala Trp Thr Gly Ser Val Asp Ile Leu Leu Glu Tyr Arg
    290                 295                 300

Ser Ser Glu Asn Pro Val Arg Tyr Gly Gln Cys Trp Val Phe Ala Gly
305                 310                 315                 320

Val Phe Asn Thr Phe Leu Arg Cys Leu Gly Ile Pro Ala Arg Ile Val
                325                 330                 335

Thr Asn Tyr Phe Ser Ala His Asp Asn Asp Ala Asn Leu Gln Met Asp
            340                 345                 350

Ile Phe Leu Glu Glu Asp Gly Asn Val Asn Ser Lys Leu Thr Lys Asp
        355                 360                 365

Ser Val Trp Asn Tyr His Cys Trp Asn Glu Ala Trp Met Thr Arg Pro
    370                 375                 380

Asp Leu Pro Val Gly Phe Gly Gly Trp Gln Ala Val Asp Ser Thr Pro
385                 390                 395                 400

Gln Glu Asn Ser Asp Gly Met Tyr Arg Cys Gly Pro Ala Ser Val Gln
                405                 410                 415

Ala Ile Lys His Gly His Val Cys Phe Gln Phe Asp Ala Pro Phe Val
            420                 425                 430

Phe Ala Glu Val Asn Ser Asp Leu Ile Tyr Ile Thr Ala Lys Lys Asp
        435                 440                 445

Gly Thr His Val Val Glu Asn Val Asp Ala Thr His Ile Gly Lys Leu
    450                 455                 460

Ile Val Thr Lys Gln Ile Gly Gly Asp Gly Met Met Asp Ile Thr Asp
465                 470                 475                 480

Thr Tyr Lys Phe Gln Glu Gly Gln Glu Glu Glu Arg Leu Ala Leu Glu
                485                 490                 495

Thr Ala Leu Met Tyr Gly Ala Lys Lys Pro Leu Asn Thr Glu Gly Val
            500                 505                 510

Met Lys Ser Arg Ser Asn Val Asp Met Asp Phe Glu Val Glu Asn Ala
        515                 520                 525

Val Leu Gly Lys Asp Phe Lys Leu Ser Ile Thr Phe Arg Asn Asn Ser
    530                 535                 540

His Asn Arg Tyr Thr Ile Thr Ala Tyr Leu Ser Ala Asn Ile Thr Phe
545                 550                 555                 560

Tyr Thr Gly Val Pro Lys Ala Glu Phe Lys Lys Glu Thr Phe Asp Val
                565                 570                 575

Thr Leu Glu Pro Leu Ser Phe Lys Lys Glu Ala Val Leu Ile Gln Ala
            580                 585                 590

Gly Glu Tyr Met Gly Gln Leu Leu Glu Gln Ala Ser Leu His Phe Phe
        595                 600                 605

Val Thr Ala Arg Ile Asn Glu Thr Arg Asp Val Leu Ala Lys Gln Lys
    610                 615                 620

Ser Thr Val Leu Thr Ile Pro Glu Ile Ile Ile Lys Val Arg Gly Thr
625                 630                 635                 640

Gln Val Val Gly Ser Asp Met Thr Val Thr Val Gln Phe Thr Asn Pro
                645                 650                 655

Leu Lys Glu Thr Leu Arg Asn Val Trp Val His Leu Asp Gly Pro Gly
            660                 665                 670

Val Thr Arg Pro Met Lys Lys Met Phe Arg Glu Ile Arg Pro Asn Ser
        675                 680                 685

Thr Val Gln Trp Glu Glu Val Cys Arg Pro Trp Val Ser Gly His Arg
    690                 695                 700

Lys Leu Ile Ala Ser Met Ser Ser Asp Ser Leu Arg His Val Tyr Gly
705                 710                 715                 720

Glu Leu Asp Val Gln Ile Gln Arg Arg Pro Ser Met
                725                 730

<210>  17
<211>  3833
<212>  DNA
<213>  Homo sapiens

<400>  17
ctttcccgag tccgtttgag gaagtccccg aggcgcacag agcaagccca cgcgagggca      60

cctctggagg ggagcgcctg caggaccttg taaagtcaaa aatgtcagaa acttccagga     120

ccgcctttgg aggcagaaga gcagttccac ccaataactc taatgcagcg gaagatgacc     180

tgcccacagt ggagcttcag ggcgtggtgc cccggggcgt caacctgcaa gagtttctta     240

atgtcacgag cgttcacctg ttcaaggaga gatgggacac taacaaggtg gaccaccaca     300

ctgacaagta tgaaaacaac aagctgattg tccgcagagg gcagtctttc tatgtgcaga 360
ttgacctcag tcgtccatat gaccccagaa gggatctctt cagggtggaa tacgtcattg 420
gtcgctaccc acaggagaac aagggaacct acatcccagt gcctatagtc tcagagttac 480
aaagtggaaa gtggggggcc aagattgtca tgagagagga caggtctgtg cggctgtcca 540
tccagtcttc ccccaaatgt attgtgggga aattccgcat gtatgttgct gtctggactc 600
cctatggcgt acttcgaacc agtcgaaacc cagaaacaga cacgtacatt ctcttcaatc 660
cttggtgtga agatgatgct gtgtatctgg acaatgagaa agaaagagaa gagtatgtcc 720
tgaatgacat cggggtaatt ttttatggag aggtcaatga catcaagacc agaagctgga 780
gctatggtca gtttgaagat ggcatcctgg acacttgcct gtatgtgatg gacagagcac 840
aaatggacct ctctggaaga gggaatccca tcaaagtcag ccgtgtgggg tctgcaatgg 900
tgaatgccaa agatgacgaa ggtgtcctcg ttggatcctg ggacaatatc tatgcctatg 960
gcgtcccccc atcggcctgg actggaagcg ttgacattct attggaatac cggagctctg 1020
agaatccagt ccggtatggc caatgctggg tttttgctgg tgtctttaac acatttttac 1080
gatgccttgg aataccagca agaattgtta ccaattattt ctctgcccat gataatgatg 1140
ccaatttgca aatggacatc ttcctggaag aagatgggaa cgtgaattcc aaactcacca 1200
aggattcagt gtggaactac cactgctgga atgaagcatg gatgacaagg cctgaccttc 1260
ctgttggatt tggaggctgg caagctgtgg acagcacccc ccaggaaaat agcgatggca 1320
tgtatcggtg tggccccgcc tcggttcaag ccatcaagca cggccatgtc tgcttccaat 1380
ttgatgcacc ttttgttttt gcagaggtca acagcgacct catttacatt acagctaaga 1440
aagatggcac tcatgtggtg gaaaatgtgg atgccaccca cattgggaaa ttaattgtga 1500
ccaaacaaat tggaggagat ggcatgatgg atattactga tacttacaaa ttccaagaag 1560
gtcaagaaga agagagattg gccctagaaa ctgccctgat gtacggagct aaaaagcccc 1620
tcaacacaga aggtgtcatg aaatcaaggt ccaacgttga catggacttt gaagtggaaa 1680
atgctgtgct gggaaaagac ttcaagctct ccatcacctt ccggaacaac agccacaacc 1740
gttacaccat cacagcttat ctctcagcca acatcacctt ctacaccggg gtcccgaagg 1800
cagagttcaa gaaggagacg ttcgacgtga cgctggagcc cttgtccttc aagaaagagg 1860
cggtgctgat ccaagccggc gagtacatgg gtcagctgct ggaacaagcg tccctgcact 1920
tctttgtcac agctcgcatc aatgagacca gggatgttct ggccaagcaa aagtccaccg 1980
tgctaaccat ccctgagatc atcatcaagg tccgtggcac tcaggtagtt ggttctgaca 2040
tgactgtgac agttcagttt accaatcctt taaaagaaac cctgcgaaat gtctgggtac 2100
acctggatgg tcctggagta acaagaccaa tgaagaagat gttccgtgaa atccggccca 2160
actccaccgt gcagtgggaa gaagtgtgcc ggccctgggt ctctgggcat cggaagctga 2220
tagccagcat gagcagtgac tccctgagac atgtgtatgg cgagctggac gtgcagattc 2280
aaagacgacc ttccatgtga atgcacagga agctgagatg aaccctggca tttggcctct 2340

tgtagtcttg gctaaggaaa ttctaacgca aaaatagctc ttgctttgac ttaggtgtga 2400
agacccagac aggactgcag agggccccag agtggagatc ccacatattt caaaaacata 2460
cttttccaaa cccaggctat tcggcaagga agttagtttt taatctctcc accttccaaa 2520
gagtgctaag cattagcttt aattaagctc tcatagctca taagagtaac agtcatcatt 2580
tatcatcaca aatggctaca tctccaaata tcagtgggct ctcttaccag ggagatttgc 2640
tcaatacctg gcctcattta aaacaagact tcagattccc cactcagcct tttgggaata 2700
atagcacatg atttgggctc tagaattcca gtcccctttc tcggggtcag gttctaccct 2760
ccatgtgaga atatttttcc caggactaga gcacaacata atttttattt ttggcaaagc 2820
cagaaaaaga tctttcattt tgcacctgca gccaagcaaa tgcctgccaa attttagatt 2880
taccttgtta gaagaggtgg ccccatatta acaaattgca tttgtgggaa acttaaccac 2940
ctacaaggag ataagaaagc aggtgcaaca ctcaagtcta ttgaataatg tagttttgtg 3000
atgcatttta tagaatgtgt cacactgtgg cctgatcagc aggagccaat atcccttact 3060
ttaacccttt ctgggatgca atactaggaa gtaaagtgaa gaatttatct ctttagttag 3120
tgattatatt tcacccatct ctcaggaatc atctcctttg cagaatgatg caggttcagg 3180
tcccctttca gagatataat aagcccaaca agttgaagaa gctggcggat ctagtgacca 3240
gatatataga aggactgcag ccactgattc tctcttgtcc ttcacatcac cattttgaga 3300
cctcagcttg gcactcaggt gctgaagggt aatatggact cagccttgca aatagccagt 3360
gctagttctg acccaaccac agaggatgct gacatcattt gtattatgtt ccaaggctac 3420
tacagagaag gctgcctgct atgtatttgc aaggctgatt tatggtcaga atttccctct 3480
gatatgtcta gggtgtgatt taggtcagta gactgtgatt cttagcaaaa aatgaacagt 3540
gataagtata ctgggggcaa aatcagaatg gaatgctctg gtctatataa ccacatttct 3600
gagcctttga gactgttcct gagccttcag cactaaccta tgagggtgag ctggtcccct 3660
ctatatatac atcatactta actttactaa gtaatctcac agcatttgcc aagtctccca 3720
atatccaatt ttaaaatgaa atgcattttg ctagacagtt aaactggctt aacttagtat 3780
attattatta attacaatgt aatagaagct taaaataaag ttaaactgat tat 3833